# EUROPEAN PATENT SPECIFICATION

(11) **EP 2 674 756 B1**
(45) Date of publication and mention of the grant of the patent: **03.08.2016**
(21) Application number: 13182442.7
(22) Date of filing: 08.09.2010
(51) Int. Cl.: G01N 33/574

(54) **Determining the expression status of human epidermal growth factor receptor 2 (HER2) in biological samples**
Bestimmung des Expressionsstatus des humanen epidermischen Wachstumsfaktorrezeptors 2 (HER2) in biologischen Proben
Détermination du statut d'expression du récepteur du facteur de croissance épidermique humain 2 (HER2) dans des échantillons biologiques

(30) Priority: 10.09.2009 US 241253 P
(43) Date of publication of application: 18.12.2013
(62) Divisional of application: 10175852.2
(73) Proprietor: Bruker Daltonik GmbH, 28359 Bremen (DE)
(72) Inventor: Albers, Christian, 28357 Bremen (DE); Belau, Eckhard, 28865 Lilienthal (DE); Deininger, Sören-Oliver, 04277 Leipzig (DE); Suckau, Detlev, 28879 Grasberg (DE); Walch, Axel, 85598 Baldham (DE); Rauser, Sandra, 81675 München (DE)
(74) Representative: Kohler Schmid Möbus Patentanwälte

(56) References cited:
- US-A1- 2008 176 258
- MACKAY A ET AL: "CDNA MICROARRAY ANALYSIS OF GENES ASSOCIATED WITH ERBB2 (HER2/NEU) OVEREXPRESSION IN HUMAN MAMMARY LUMINAL EPITHELIAL CELLS", ONCOGENE, NATURE PUBLISHING GROUP, GB, vol. 22, no. 17, 1 May 2003 (2003-05-01), pages 2680-2688, XP009018980, ISSN: 0950-9232, DOI: 10.1038/SJ.ONC.1206349
- HAO JIHUA ET AL: "Identification and rational redesign of peptide ligands to CRIP1, a novel biomarker for cancers", PLOS COMPUTATIONAL BIOLOGY, PUBLIC LIBRARY OF SCIENCE, US, vol. 4, no. 8, 1 August 2008 (2008-08-01), pages E1000138-1, XP002615159, ISSN: 1553-734X, DOI: 10.1371/JOURNAL.PCBI.1000138
- MA X-J ET AL: "Gene expression profiles of human breast cancer progression", PROCEEDINGS OF THE NATIONAL ACADEMY OF SCIENCES - PNAS, NATIONAL ACADEMY OF SCIENCES, US, vol. 100, no. 10, 13 May 2003 (2003-05-13) , pages 5974-5979, XP002615160, ISSN: 0027-8424, DOI: 10.1073/PNAS.0931261100 [retrieved on 2003-04-24]
- LIU SHUQIAN ET AL: "Thiamine transporter gene expression and exogenous thiamine modulate the expression of genes involved in drug and prostaglandin metabolism in breast cancer cells", MOLECULAR CANCER RESEARCH, AMERICAN ASSOCIATION FOR CANCER RESEARCH, US, vol. 2, no. 8, 1 August 2004 (2004-08-01), pages 477-487, XP002615161, ISSN: 1541-7786

## Description

### Background of the Invention

Adenocarcinoma of the breast is a leading cause of cancer morbidity and mortality among women worldwide. A major challenge faced by clinicians treating patients with breast cancer is how to best assess patient outcome and predict the clinical course of the disease so that the most appropriate treatment regimen can be identified. Determining the expression status of the human epidermal growth factor receptor 2 (HER2) in newly diagnosed breast cancer tissues is critically important for therapeutic decision making. Current guidelines recommend that the HER2 expression status should be evaluated for every patient with newly diagnosed, primary breast cancer. Unlike most histopathologic testing, which serves as an adjunct to establishing a diagnosis, the results of HER2 testing stand alone in determining which patients afflicted with primary breast cancer are likely to respond to trastuzumab (Herceptin^{®}), a monoclonal antibody directed to HER2. The HER2 expression status can be determined at the DNA-, the mRNA-, or the protein level. Various techniques are available for assessing these different target molecules, each with benefits and drawbacks. Although almost a decade has passed since trastuzumab was initially approved by the USA Food and Drug Administration (FDA), accurate HER2 expression status determination continues to challenge the field of clinical laboratory testing. Currently, two testing methods are approved by the FDA for HER2 testing of breast cancer tissues in the laboratory: immunohistochemical analysis (IHC) and fluorescence in situ hybridization (FISH).

There are previous findings using gene transcription profiles that show an increased transcription of the gene related to the human cystein-rich intestinal protein 1 (CRIP1) in human breast cancers compared to non-diseased tissues (Ma et al., 2003; Liu et al., 2004) and other cancer types. In experiments comparing transcription of the CRIP1 gene in human breast cancer to matched normal breast tissue, the mRNA for this target was over-expressed 8 to 10-fold (Ma et al., 2003). Two recent studies compared differential gene transcription patterns in HER2-positive and HER2-negative breast cancer cell lines and tissues (Wilson et al., 2002; Mackay et al., 2003), wherein the mRNA of the CRIP1 gene and HER2-positive were found to be both up-regulated. In contrast to these genetic findings, an increased expression of the CRIP 1 at the protein level has not been shown yet.

CRIP1 belongs to the LIM/double zinc finger protein family, which includes cysteine-and glycine-rich protein-1, rhombotin-1, rhombotin-2, and rhombotin-3. Human CRIP1, primarily a cytosolic protein, was cloned in 1997 using RT-PCR of human small intestine RNA and oligonucleotides whose sequence was derived from the human heart homologue of this protein, CRHP.

The following four publications are also dealing with analytical methods and detailed aspects in the context of cancer cell detection, in particular with predicting the response of a cancer patient to a drug, predicting the prognosis of a cancer patient and for diagnosing cancer in a subject:
- D1: MACKAY A ET AL: "CDNA MICROARRAY ANALYSIS OF GENES ASSOCIATED WITH ERBB2 (HERZ/NEU) OVEREXPRESSION IN HUMAN MAMMARY LUMINAL EPITHELIAL CELLS", ONCOGENE, NATURE PUBLISHING GROUP, GB, vol. 22, no. 17, 1 May 2003 (2003-05-01), pages 2680-2688, XP009018980
- D2: HAO JIHUA ET AL: "Identification and rational redesign of peptide ligands to CRIP1, a novel biomarker for cancers", PLOS COMPUTATIONAL BI-OLOGY, PUBLIC LIBRARY OF SCIENCE, US, vol. 4, no. 8, 1 August 2008 (2008-08-01), pages El 000138-1, XP002615159
- D3: MA X-J ET AL: "Gene expression profiles of human breast cancer progression", PROCEEDINGS OF THE NATIONAL ACADEMY OF SCIENCES - PNAS, NATIONAL ACADEMY OF SCIENCES, US, vol. 100, no. 10, 13 May 2003 (2003-05-13), pages 5974-5979, XP002615160
- D4: LIU SHUQIAN ET AL: "Thiamine transporter gene expression and exogenous thiamine modulate the expression of genes involved in drug and prostaglandin metabolism in breast cancer cells", MOLECULAR CANCER RESEARCH, AMERICAN ASSOCIATION FOR CANCER RESEARCH, US, vol. 2, no. 8, 1 August 2004 (2004-08-01), pages 477-487, XP002615161

However, none of above cited four publications discloses nor suggests variants of the Cystein-rich intestinal protein 1 (CRIP1) having one of following molecular weights 8419, 8435, 8450 or 8465 atomic mass units.

Mass spectrometry has extensively been used for investigating protein patterns in biological samples, like body fluids or homogenized tissue, to find biomarkers. Interrogation of the resulting complex mass spectrometry (MS) data sets using modern computational tools has resulted in identification of both disease-state and patient-prognosis specific protein patterns. In general, biomarkers are used or at least predicted to be used for diagnosing cancer, qualifying different types of cancer, predicting the response to a cancer drug or predicting the prognosis of a cancer patient. Biomarkers can further be measured and optionally be identified with high accuracy by tandem mass spectrometry, e.g. by multiple reaction monitoring (MRM).

Matrix-assisted laser desorption/ionization (MALDI) imaging mass spectrometry (IMS) is a powerful tool for investigating protein patterns through the direct (in situ) analysis of tissue sections. Similarly to IHC and FISH, MALDI IMS has advantage over other assay methods (i.e. those requiring homogenization), because it is morphologically driven. This allows the direct evaluation of tumor cells, correlation with other morphologic features and the ability to assay smaller patient tumor tissue specimen such as needle core biopsy specimens. This makes it a seemingly ideal tool for rapid tissue diagnostics and molecular histology. Besides, MALDI IMS can determine the distribution of hundreds of compounds in a single measurement without any need of labeling.

Previously published work using MALDI IMS for tumor classification suggests that this technology enables accurate classification (Yanagisawa et al., 2003; Schwartz et al., 2005). To date, MALDI IMS has been applied to multiple types of diseased tissues, including human non-small cell lung tumors, gliomas, as well as ovarian and breast cancers. From recent publications it has become clear that the integration of MALDI IMS into clinical management regarding disease diagnosis and outcome prediction will take place soon (Franck et al., 2009; Cornett et al., 2007). As MALDI IMS protein expression data will become available for various tumor tissue types, this approach will provide a common disease-wide methodology that can be applied to a variety of clinical questions. While many of these MALDI IMS studies rather focus on the identification of new biomarkers, so far only few studies have evaluated the potential of MALDI IMS for molecular classification of tissues based on protein patterns.

### Summary of the Invention

1. In a first aspect, the present invention provides a method for either predicting the response of a cancer patient to a drug, or predicting the prognosis of a cancer patient, comprising:
   (a) measuring amounts of one or more variants of the Cystein-rich intestinal protein 1 (CRIP1 variants) in a sample of the patient wherein the variants have one of following molecular weights 8419, 8435, 8450 or 8465 atomic mass units, and
2. comparing the amounts of the one or more variants to control amounts determined from samples of either patients responding and not responding to the drug, respectively, or patients with different prognosis.

In a second aspect, the present invention provides a method for diagnosing cancer in a subject comprising:
(a) measuring the amounts of one or more variants of the Cystein-rich intestinal protein 1 (CRIP1 variants)in a liquid sample of the subject selected from the group comprising serum, plasma, whole blood, nipple aspirate fluid, cerebrospinal fluid and urine, wherein the variants have one of following molecular weights 8419, 8435, 8450 or 8465 atomic mass units, and
comparing the amounts of the one or more variants to control amounts determined from samples of healthy subjects.

In a preferred embodiment, the above defined methods according to the invention can be modified in that the amounts of the variants are either measured by mass spectrometry or are measured and optionally identified by tandem mass spectrometry.

This embodiment can be further improved in that the one or more variants are extracted from the sample between steps (a) and (b) utilizing an antibody or other affinity reagents, like aptamers or affibodies, specific for the human cystein-rich intestinal protein 1 or its variants.

A further preferred embodiment of the above defined methods according to the invention is characterized in that the variants of the human cystein-rich intestinal protein 1 either are modified by one or more structural variations, such as at least one of disulfide bond formation, one or more methyl groups, and oxygen atoms, or are modified by at least one mutation.

### Description of the Drawings

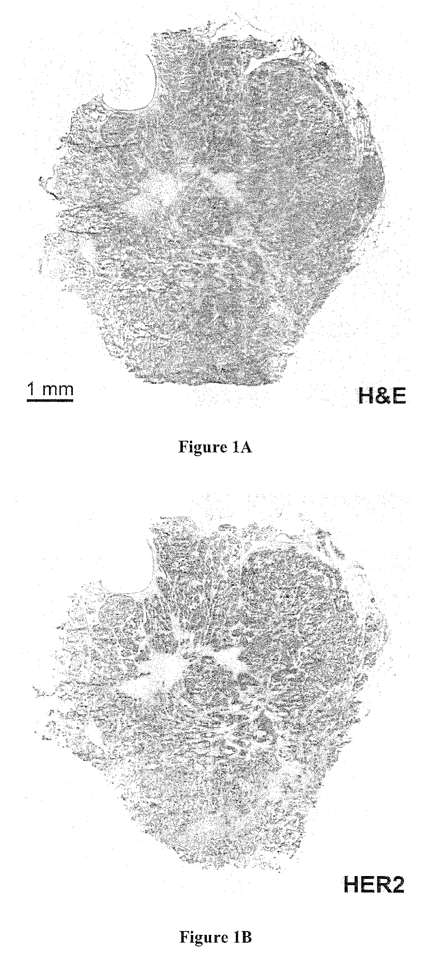

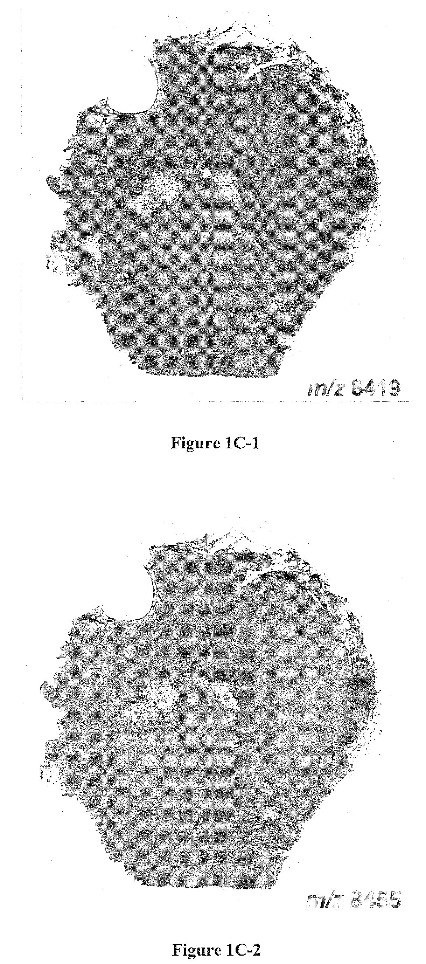

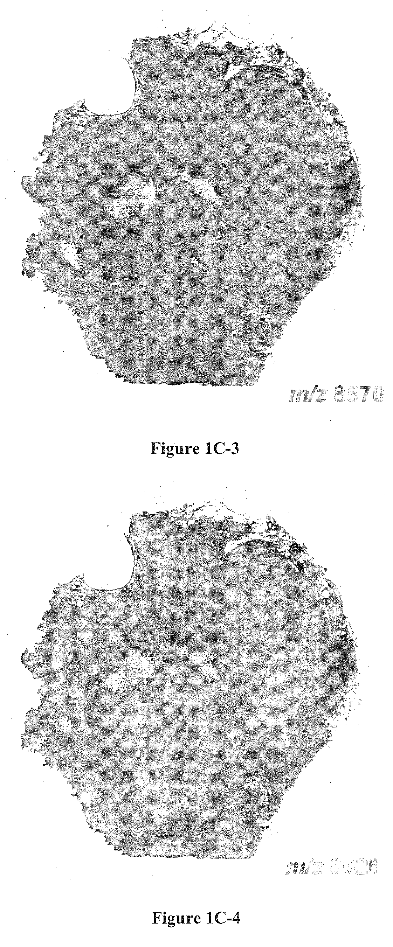

**Figure 1** MALDI IMS of a HER2-positive breast cancer tissue section. A Optical microscopic image of the hematoxylin and eosin (H&E) stained tissue section. The staining was done following the MALDI measurement of the very same tissue section. B Immunohistochemistry for HER2 in a serial section of the breast cancer sample, which is classified as score 3+. **C-1** to **C-4** Overlay of aligned H&E (A) and MALDI images. Four representative m/z species which are only present in HER2-positive tumors are selected for visualization of ion density images (m/z 8419, m/z 8455, m/z 8570, m/z 8626). These features are tumor cell specific and are not present in other cell types (e.g. stromal tissue). The Scale bar is 1mm.

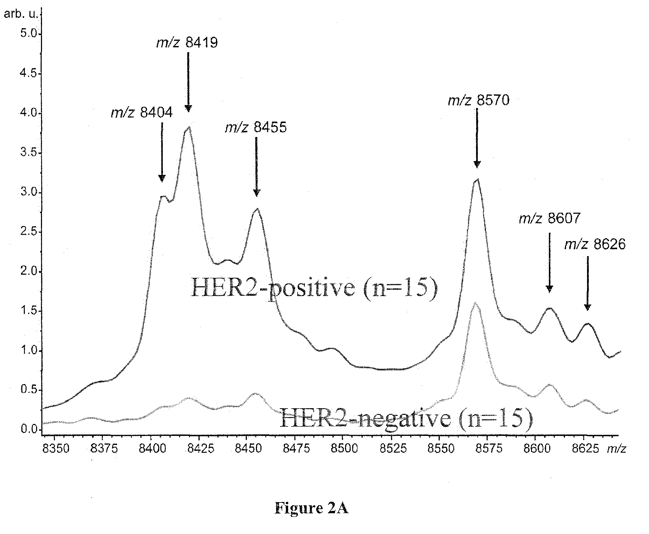

**Figure 2A** Examples of averaged MALDI-TOF MS spectra of HER2-positive (n=15) and HER2-negative (n=15) breast cancer tissues (discovery set) in the mass range *of m*/*z* 8345 to 8640. The arrows mark six peaks with significant differences between the HER2-positive and HER2-negative groups with centroid masses at *m*/*z* 8404, 8419, 8455, 8570, 8607, and 8626.

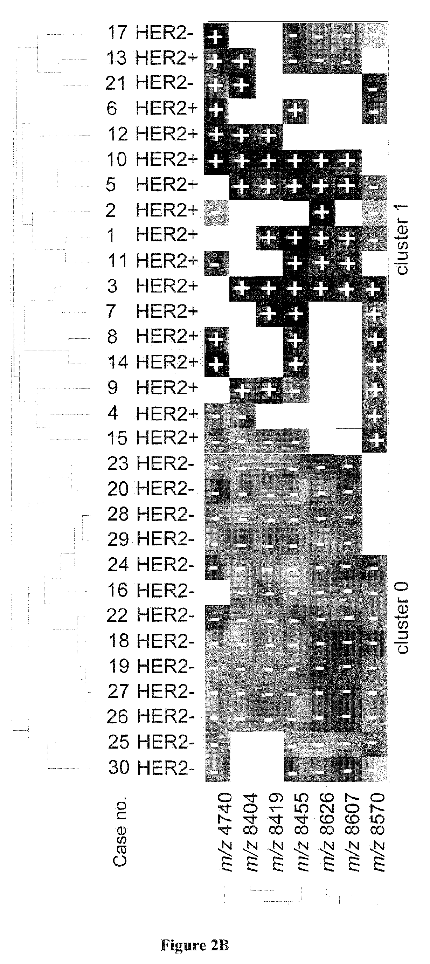

**Figure 2B** Hierarchical clustering of breast cancer tumor tissue specimens from the discovery set. All samples were clustered according to their expression pattern of the seven discriminating peptide ions at *m*/*z* 4740, 8404, 8419, 8455, 8570, 8607, and 8626. Peak intensities were standardized before clustering. Over-expressed peptide signals are shown as plus signs, under-expressed as minus signs and unchanged ones blank. The algorithm identified two clusters (shown as cluster 0 and cluster 1). While cluster 0 harbors only HER2-negative samples (n=13), two of the HER2-negative cases (17 and 21) were assigned as false positives to cluster 1 (HER2-positive group).

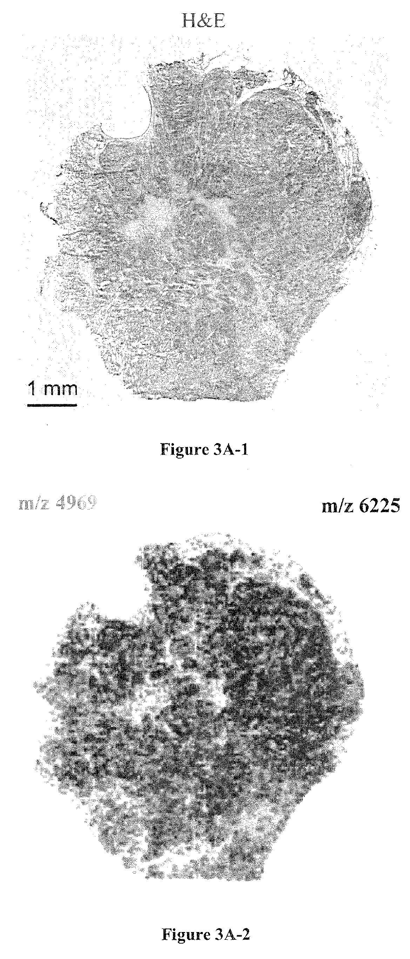

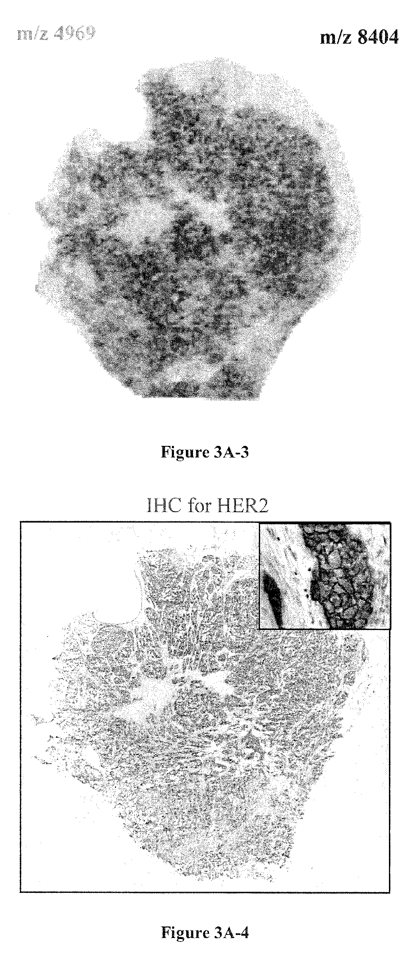

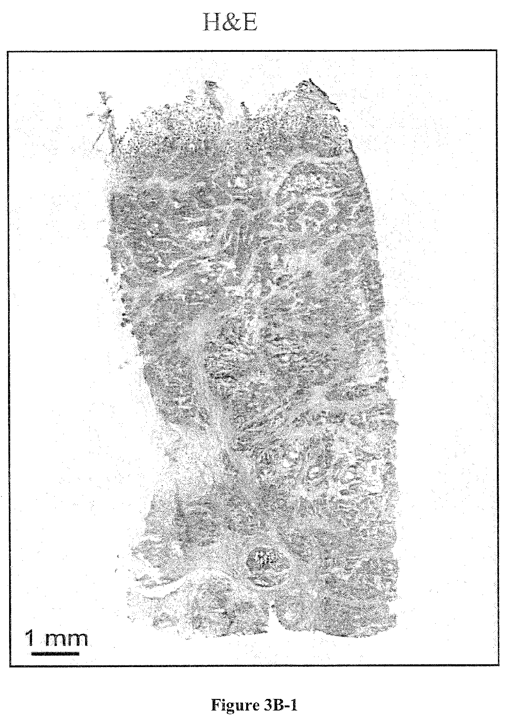

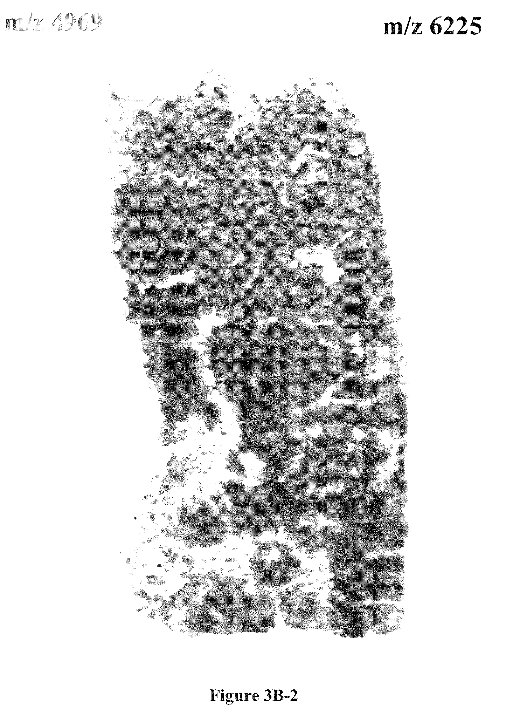

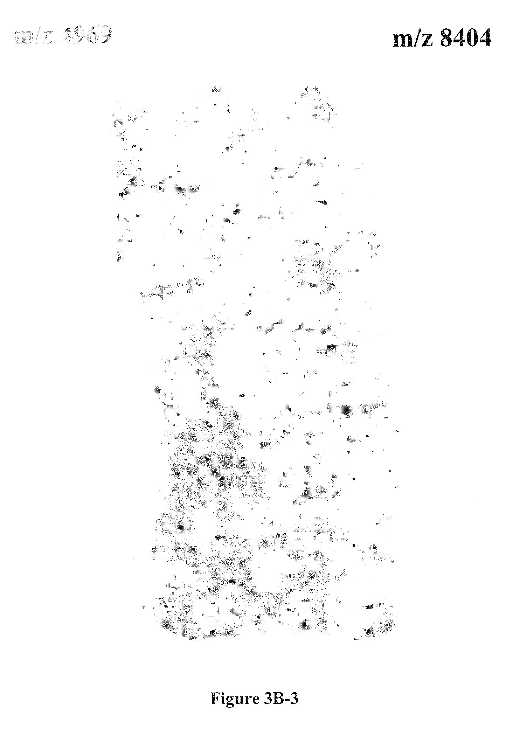

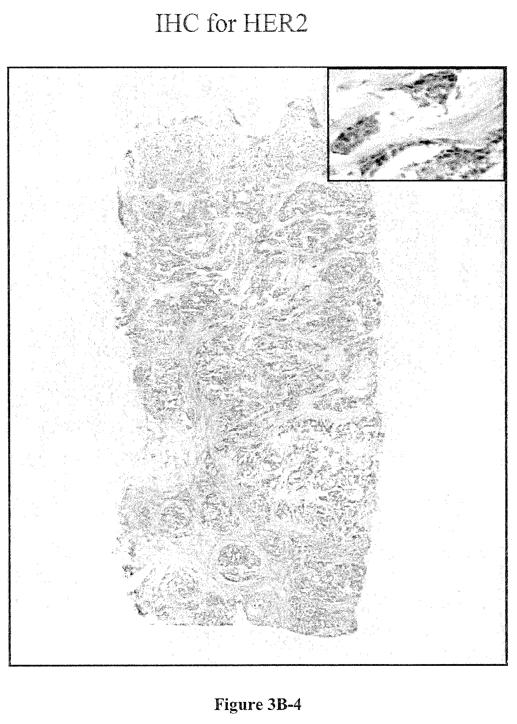

**Figure 3** Visualization of *m*/*z* 8404 as an example of a highly up-regulated spectral feature in HER2-positive breast cancer tissues: Optical microscopic images of the MALDI IMS measured and subsequently H&E stained original tissue sections of a HER2-positive case (Fig. 3A-1) and a HER2-negative case (Fig. 3B-1). Immunohistochemistry for HER2 of the respective case on a consecutive serial section are shown in Fig. 3A-4 and Fig. 3B-4, respectively.
Note the strong immunoreactivity (score 3+) of the positive case while the reaction in the negative case indicates a score 0. The visualization of the *m*/*z* 8404 (Fig. 3A-3, Fig. 3B-3) found by MALDI IMS as highly up-regulated in HER2-positive tissues (Fig. 3A-3) clearly shows that this spectral feature is specific for cancer cells in this HER2-positive case but absent in the HER2-negative case (Fig. 3B-3). The spectral feature of *m*/*z* 6225 (Fig. 3A-2, Fig. 3B-2) is an example specific for cancer cells but not distinguishing between HER2-positive and HER2-negative tissues and is therefore present in both cases. *m*/*z* 4969 is a *m*/*z* species specific for tumor stroma and thus also present in both cases.

The preferred variant describes the MALDI IMS technology for determining the HER2 expression status in breast cancer tissues. Clinical laboratory testing for HER2 expression status in newly diagnosed, primary breast cancer tissues is critically important for therapeutic decision making. Matrix-assisted laser desorption/ionization (MALDI) imaging mass spectrometry (IMS) is a powerful tool for investigating proteins through the direct analysis of tissue sections. Unlike immunohistochemistry (IHC), MALDI-IMS enables the acquisition of complex protein expression profiles without any labeling.

Sample collection and tumor tissue specimens: Primary breast cancer patients presenting with invasive ductal carcinoma underwent primary surgical resection at the Department of Obstetrics and Gynecology, Klinikum rechts der Isar, Technische Universität München. Tumor tissue samples were snap-frozen and stored in liquid nitrogen in the tissue bank of the Institute of Pathology, Technische Universität München. All tumor tissue specimens were procured from patients giving written informed consent. The study was approved by Ethics Committee of the Technische Universität München. A total of 48 patients (30 for discovery set and 18 for validation set) were recruited for this study. The histomorphological and clinical characteristics of the analyzed cases are listed in Table 1.

Assessment of HER2 expression status: All cases were carefully reviewed before study inclusion for HER2 expression status. The discovery set was evaluated by immunohistochemistry (IHC) and fluorescence in situ hybridization (FISH), while the validation set was evaluated by IHC only. Scoring criteria were applied according to the American Society of Clinical Oncology/College of American Pathologists guideline recommendations for HER2 testing in breast cancer (Wolff et al., 2007). A positive HER2 result is IHC staining of 3+ (uniform, intense membrane staining of > 30% of invasive tumor cells), a FISH result of more than six HER2 gene copies per nucleus or a FISH ratio (HER2 gene signals to chromosome 17 signals) of more than 2.2; a negative result is an IHC staining of 0 or 1+, a FISH result of less than 4.0 HER2 gene copies per nucleus or FISH ratio of less than 1.8. In total, 48 frozen tissue samples of invasive ductal breast cancer patients were used and the pre-characterized HER2 expression status was confirmed again by IHC on a consecutive cryosection.

MALDI Imaging Mass Spectrometry (MALDI IMS) experiments: Samples were cryo-sectioned on conductive Indium-Tin-Oxide (ITO) coated glass slides (Bruker Daltonik GmbH, Bremen, Germany), briefly washed in 70% and 100% ethanol, dried under vacuum, and directly covered with matrix. The MALDI matrix was applied by use of the ImagePrep station (Bruker Daltonik GmbH, Bremen, Germany) and the standard protocol provided with the instrument, whereas sinapinic acid (Sigma-Aldrich, Taufkirchen, Germany) at 10 mg/ml in water/acetonitrile 40:60 (v/v) with 0.2% trifluoroacetic acid (TFA, Applied Biosystems, Darmstadt, Germany) was used as matrix for the MALDI measurements. The MALDI measurement was performed on an Ultraflex III MALDI-TOF/TOF in linear mode with a mass range of 2400-25000 u with a sampling rate of 0.1 GS/s using FlexControl 3.0 and FlexImaging 2.1 software packages (all Bruker Daltonik GmbH, Bremen, Germany). The lateral resolution for MALDI IMS was set to 200 µm and a total of 200 laser shots were accumulated per pixel at constant laser power.

Following the MALDI IMS experiments, the cryosections were incubated in 70% ethanol to remove the matrix and then washed in distilled water. Subsequently, the very same section, which was measured by MALDI IMS, was stained with hematoxylin and eosin (H&E), scanned with the MIRAX^{®} DESK system (Carl Zeiss MicroImaging GmbH, Göttingen, Germany), and co-registered with the MALDI IMS results to correlate mass spectrometric data with the histological features of the section.

Statistical analyses: Tumor associated spectra were selected using the FlexImaging 2.1 software (Bruker). For this purpose, we defined in every tissue section regions of interest (ROIs) that contained invasive ductal breast cancer tissue specific for HER2-positive and HER2-negative cell populations, respectively. These extracted mass spectra comprising about 300 peaks per spectrum underwent recalibration on common "background" peaks (also known as spectral alignment) and normalization based on their total ion count in the observation mass range utilizing ClinProTools 2.2 software (Bruker Daltonik GmbH, Bremen, Germany). An average spectrum across all spectra of an ROI was used for peak defining integration ranges. These integration ranges were used to obtain the average peak intensities of each respective ROI. Significant differences in peak intensities between both histological groups (HER2-positive and HER2-negative) were evaluated by the Wilcox on rank-sum test with a significance cutoff point of p < 0.05.

For classification and further statistical analyses, data were exported to the R statistical software (R Foundation for Statistical Computing) in order to compare the quality of two different classification algorithms predicting HER2 expression status. For this purpose, a support vector machine (SVM) and an artificial neuronal network (ANN) were computed on the discovery set of samples. Both classifiers were tested internally and externally using a 10-fold cross validation and the validation set, respectively. At each evaluation step the features were selected that best segregated the samples within the training set into the related groups (i.e. HER2-positive or HER2-negative), where an MS signal was included once its area under curve (AUC) exceeded 0.8 as calculated by receiver operating characteristics (ROC) analysis. Finally, the performances of both approaches were compared by analyzing the resulting confusion matrices across all evaluation steps.

The additional hierarchical clustering of the expression profiles of all samples was carried out with the software CLUTO (George Karypis, Department of Computer Science & Engineering, University of Minnesota, USA) on the standardized, exported data. CLUTO is a software package for clustering low- and high-dimensional datasets and for analyzing the characteristics of the various clusters.

Results: In the initial discovery experiment, we analyzed 30 tumor tissue samples (15 HER2-negative samples, 15 HER2-positive samples). 1129 individual mass spectra, representing specific HER2-positive or HER2-negative tumor cell populations defined by ROIs, were used for comparative analyses. Representative examples of images derived from mapping discriminating peaks are shown in Figures 1 and 3. Another peak at *m*/*z* 4969 was expressed in tumor stroma with little or no expression seen in cancer cells (Figures 3A-2, 3A-3, 3B-2 and 3B-3). In the mass range *m*/*z* 8345 to 8640, five peptide ions could be detected, which showed an over-expression in the HER2-positive cases compared to the HER2-negative cases (Figures 1C-1 to 1C-4 and 2A). In total, seven *m*/*z* species were found at an average of *mlz* 4740, *m*/*z* 8404, *mlz* 8419, *mlz* 8455, *m*/*z* 8570, *mlz* 8607 and *mlz* 8626, which discriminate between the HER2-positive and the HER2-negative tumor samples. These peaks were clearly different from the background noise and were determined with a mass accuracy of ±4 u average molecular weight in linear mode. Besides the above MS signals, there is another differentiating MS signal (not shown in the Figures 1 to 3) at about m/z 22490 being specific for invasive ductal cancer cells but not present in tumor stroma or other tissue components.

According to the expression pattern of the above described seven m/z species, a hierarchical clustering was performed on the samples of the discovery set (Figure 2B). The algorithm identified two clusters. While one cluster harbors HER2-negative samples (n=13), only two HER2-negative samples (case numbers 17 and 21) were assigned as false-positives to the HER2-positive designated group.

For performance assessment of both classifiers (SVM and ANN) first a 10-fold cross-validation was applied on the sample data (discovery set). The ANN outperformed the SVM by gaining a correct classification rate of 90% allowing a reliable discrimination of the HER2-positive and HER2-negative groups. The classification ability of both classifiers (SVM and ANN) was furthermore used to predict the HER2 expression status of the tissues from the validation set (n=18). This resulted in a misclassification of two cases. Therefore both approaches achieved an overall accuracy of 89%, with a sensitivity of 83% and a specificity of 92% (Table 1).

Identification of one of the marker proteins by tissue lysis and tandem mass spectrometric analysis: Two human HER2-positive breast cancer biopsy slices (10 µm thickness, not previously stained or analyzed by MALDI imaging) were placed in an 1.5 ml Eppendorf tube with 50 µl 0.1% TFA followed by a 2 min centrifugation (20,000 g) to spin down the tissue (Eppendorf Centrifuge 5804 R, Eppendorf, Germany). To lyse the tissue, ultrasonication in an ice bath was applied for 30 min. Subsequently, centrifugation (20,000 g) was performed at 4°C for 30 min. Tissue lysate was further processed with ultrafiltration (Vivaspin 500, cutoff 5 ku, Sartorius Stedim Biotech, France). 25 µl of supernatant from centrifugation was pipetted into a spin column and centrifuged for one hour at 15,000 g. To wash the lysate, 25 µl 0.1%TFA was added to the spin column and centrifuged again for one hour. Spin column was refilled to 25 µl (0.1% TFA), the membrane was rinsed with this solution to obtain proteins and the solution filled into an auto sampler vial. Intact proteins were then separated with an Agilent mRP column (5 µm, 0.5 x 100 mm) and Agilent 1200 HPLC system (Agilent Technologies, Böblingen, Germany). Following LC parameters were used: Flow rate 12 µl/min, column oven set to 60°C, injection volume 8 µl, solvent A 0.1% formic acid (Sigma-Aldrich) in water, solvent B 0.1% formic acid in acetonitrile (ACN, Sigma-Aldrich), step wise gradient 7 min at 3% B, in 23 min to 22% B, in 5 min to 38% B, in 2 min to 97% B, 5 min at 97% B, in 2 min to 3% B, and 5 min at 3% B. Fractionation was performed with a Proteineer fc (Bruker Daltonik GmbH, Bremen, Germany) from 10-42 min with 20 sec time slices into a 96 well plate; two separations were collected in one well plate. For candidate localization 0.5 µl from collected fractions were spotted onto 96PAC target and analyzed with an Ultraflex III MALDI-TOF/TOF in linear mode (both: Bruker Daltonik GmbH, Bremen, Germany).

For ETD/PTR fragmentation three subsequent fractions containing the candidate peptides were combined (approx. 15 µl) and diluted with 1.5 µl 2-propanol (Sigma-Aldrich) and 7.5 µl ACN/ H₂O with 0.2% formic acid. Infusion MS experiments were performed with TriVersa NanoMate (10 µl sample, 384 well plate, D-chip with 0.4 psi and 1.55 kV; Advion Biosystems, Ithaca, NY, USA) and amaZon ETD Ion Trap (Bruker Daltonik GmbH, Bremen, Germany) as described previously (Hartmer et al., 2008). Briefly, the sample was measured with Maximum Scan Mode at 4600/sec, polarity positive, Scan Range from *mlz* 100-3000, 10 spectra averaged and rolling average of 4. Chosen precursor was *m*/*z* 602.4 (charge state 14⁺) with analyte ICC of 125,000 and isolation width of *m*/*z* 4.0, MS² stage was ETD with reaction time of 45 ms and reactant ICC of 115,000, MS³ stage was PTR with reaction time of 80 ms and reactant ICC of 175,000. Acquired ETD/PTR spectra were processed using DataAnalysis 4.0 with the SNAPII algorithm, deconvoluted spectra were further analyzed with BioTools 3.2 (all Bruker Daltonik GmbH, Bremen, Germany) and submitted to a Mascot (Matrix Science, London, UK) database search (Mascot 2.2.04, SwissProt 56.1, taxonomy: mammals, Peptide mass tolerance +/- 10 u, fragment mass tolerance +/- 0.8 u, Instrument type ETD-Trap). The identified protein sequence was manually validated in BioTools on a residue-by residue basis using the raw data.

Cystein-rich protein 1 (CRIP1_HUMAN) was identified by database searching with a mascot score of 126. A theoretical average molecular weight of 8402 u was calculated from the underivatized CRIP1_HUMAN des-Met 1 gene sequence in good agreement with the MALDI and ESI data. However, a cluster of CRIP1 variants at 8419, 8435, 8450 and 8464 u were determined experimentally after charge deconvolution of the multiple charge states in the ion trap ESI spectrum. The varying molecular weights result from one or more structural variations, wherein the human cystein-rich intestinal protein 1 is modified, e.g., by disulfide bond formation and/or one or more methyl groups and/or oxygen atoms and/or by at least one mutation.

The MALDI IMS proteomic approach, as one preferred embodiment described above, is a label-free and morphology-driven alternative for HER2 testing in breast cancer tissues and will have great implications for clinical management of breast cancer patients, and may be applicable to other cancer diseases as well. Only small amounts of fresh-frozen and unprocessed tumor tissue, easily available in a clinical setting, are needed to obtain the profiles of the marker proteins that can be used to accurately classify tumors associated with molecular features such as HER2. The tissue sample can be gained from core needle biopsies or can be available as a tissue microarray set.

Beyond classification of HER2 expression status, some of the differentially expressed species may be, directly or indirectly, responsible for the more aggressive properties of HER2 over-expressing cancer cells. In addition to defining new diagnostic and prognostic markers, this approach may also result in finding new therapeutic targets and can be used to allocate patients with small tumors to good or poor prognostic groups, and to predict response to endocrine therapy (e.g. tamoxifen or aromatase inhibitors) or to immunotherapy with trastuzumab.

Finally, one must take into consideration that the MS technology for imaging of tissue biopsies is just starting to be used in clinical research environments. One can expect that significant advances in sensitivity, speed, resolution, and biocomputational aspects of this technology will further focus on the application in diagnostic pathology.

### References

Cornett DS, Reyzer ML, Chaurand P, Caprioli RM: MALDI imaging mass spectrometry: molecular snapshots of biochemical systems. Nat Methods. 2007;4(10):828-33.
Franck J, Arafah K, Elayed M, Bonnel D, Vergara D, Jacquet A, Vinatier D, Wisztorski M, Day R, Fournier I, Salzet M: MALDI IMAGING: State of the art technology in clinical proteomics. Mol Cell Proteomics. 2009 May 18
Liu S, Stromberg A, Tai H, Moscow JA: Thiamine transporter gene expression and exogenous thiamine modulate the expression of genes involved in drug and prostaglandin metabolism in breast cancer cells. Mol Cancer Res. 2004;2:477-487.
Ma XJ, Salunga R, Tuggle JT, Gaudet J, McQuary P, et al: Gene expression profiles of human breast cancer progression. Proc Natl Acad Sci USA. 2003;100:5974-5979.
Mackay A, Jones C, Dexter T, Silva RL, Bulmer K, Jones A, Simpson P, Harris RA, Jat PS, Neville AM, Reis LF, Lakhani SR, O'Hare MJ: cDNA microarray analysis of genes associated with ERBB2 (HER2/neu) overexpression in human mammary luminal epithelial cells. Oncogene. 2003;22(17):2680-8.
Schwartz SA, Weil RJ, Thompson RC, Shyr Y, Moore JH, Toms SA, Johnson MD, Caprioli RM: Proteomic-based prognosis of brain tumor patients using direct-tissue matrix-assisted laser desorption ionization mass spectrometry. Cancer Res. 2005 Sep 1;65(17):7674-81.
Wilson KS, Roberts H, Leek R, Harris AL, Geradts J: Differential gene expression patterns in HER2/neu-positive and -negative breast cancer cell lines and tissues. Am J Pathol. 2002;161(4):1171-85.
Yanagisawa K, Shyr Y, Xu BJ, Massion PP, Larsen PH, White BC, Roberts JR, Edgerton M, Gonzalez A, Nadaf S, Moore JH, Caprioli RM, Carbone DP: Proteomic patterns of tumour subsets in non-small-cell lung cancer, The Lancet, Volume 362, Issue 9382, 9 August 2003, Pages 433-439

**Table 1**

| Clinical and molecular characteristics of the patient series | | |
|---|---|---|
| Set characteristics | Discovery set | Validation set |
| Patients | 30 | 18 |
| Mean age (range) | 60.1 (38-91) | 61.4 (36-84) |
| HER2 expression status | | |
| IHC | | |
| Score 3+ | 13 | 6 |
| Score 2+ | 2 | 0 |
| Score 1+ | 2 | 6 |
| Score 0 | 13 | 6 |
| FISH | | |
| positive | 15 | n.a. |
| negative | 15 | n.a. |
| pT classification | | |
| pT1mic | 0 | 1 |
| pT1a | 2 | 0 |
| pTlb | 1 | 0 |
| pT1c | 9 | 0 |
| pT2 | 16 | 11 |
| pT3 | 1 | 1 |
| pT4 | 1 | 0 |
| pTx | 0 | 5 |
| pN classification | | |
| positive | 17 | 6 |
| negative | 12 | 7 |
| pNx | 1 | 5 |
| Grading | | |
| Grade 1 | 0 | 0 |
| Grade 2 | 3 | 6 |
| Grade 3 | 27 | 12 |
| ER / PR status | | |
| ER+ | 14 | 5 |
| ER- | 16 | 13 |
| PR+ | 9 | 13 |
| PR- | 21 | 5 |
| | | |

| Proteomic classification | Discovery set* | Validation set |
|---|---|---|
| Support Vector Mach ine | | |
| Accuracy | 87% | 89% |
| | (95% CI 70% - 96%) | (95% CI 65% - 99%) |
| Sensitivity | 87% | 83% |
| Specificity | 87% | 92% |
| | | |
| Artificial Neuronal Network | | |
| Accuracy | 90% | 89% |
| | (95% CI 73% - 98%) | (95% CI 65% - 99%) |
| Sensitivity | 87% | 83% |
| Specificity | 93% | 92% |

| | | |
|---|---|---|
| Abbreviations used in Table 1: HER2 = human epidermal growth factor receptor 2, IHC = immunohistochemistry, FISH = *fluorescence in situ* hybridization, ER = estrogen receptor, PR = progesterone receptor, CI = confidence interval, n.a. = not analyzed * Classifiers were evaluated using a 10-fold cross-validation | | |

## Claims

1. A method for either predicting the response of a cancer patient to a drug, or predicting the prognosis of a cancer patient, comprising:
(a) measuring amounts of one or more variants of the Cystein-rich intestinal protein 1 (CRIP1 variants) in a sample of the patient wherein the variants have one of following molecular weights 8419, 8435, 8450 or 8465 atomic mass units, and
(b) comparing the amounts of the one or more variants to control amounts determined from samples of either patients responding and not responding to the drug, respectively, or patients with different prognosis.

2. A method for diagnosing cancer in a subject comprising:
(a) measuring the amounts of one or more variants of the Cystein-rich intestinal protein 1 (CRIP1 variants)in a liquid sample of the subject selected from the group comprising serum, plasma, whole blood, nipple aspirate fluid, cerebrospinal fluid and urine, wherein the variants have one of following molecular weights 8419, 8435, 8450 or 8465 atomic mass units, and
(b) comparing the amounts of the one or more variants to control amounts determined from samples of healthy subjects.

3. The method of claim 1 or 2, wherein the amounts of the variants are either measured by mass spectrometry or are measured and optionally identified by tandem mass spectrometry.

4. The method of claim 3, wherein the one or more variants are extracted from the sample between steps (a) and (b) utilizing an antibody or other affinity reagents, like aptamers or affibodies, specific for the human cystein-rich intestinal protein 1 or its variants.

5. The method of claim 1 or 2, wherein the variants of the human cystein-rich intestinal protein 1 either are modified by one or more structural variations, such as at least one of disulfide bond formation, one or more methyl groups, and oxygen atoms, or are modified by at least one mutation.

## Patentansprüche

1. Verfahren entweder zum Vorhersagen der Reaktion eines Krebspatienten auf ein Medikament oder zum Vorhersagen der Prognose eines Krebspatienten, umfassend:
(a) Messen der Mengen von einer oder mehreren Varianten des Cystein-reichen Darmproteins 1 (CRIP1 Varianten) in einer Probe des Patienten, wobei die Varianten eines der folgenden Molekulargewichte von 8419, 8435, 8450 oder 8465 atomaren Masseneinheiten haben, und
(b) Vergleichen der Mengen einer oder mehrerer Varianten mit Kontrollmengen, die aus Proben von Patienten bestimmt wurden, die entweder auf das Medikament ansprachen bzw. nicht auf das Medikament ansprachen, oder von Patienten mit einer anderen Prognose.

2. Verfahren zum Diagnostizieren von Krebs in einem Subjekt, umfassend:
(a) Messen der Mengen von einer oder mehreren Varianten des Cystein-reichen Darmproteins 1 (CRIP1 Varianten) in einer flüssigen Probe des Subjektes, ausgewählt aus der Gruppe umfassend Serum, Plasma, Vollblut, Nippelaspiratflüssigkeit, Cerebrospinalflüssigkeit und Urin, wobei die Varianten eines der folgenden Molekulargewichte haben: 8419, 8435, 8450 oder 8465 atomaren Masseneinheiten, und
(b) Vergleichen der Mengen der einen oder mehreren Varianten mit Kontrollmengen, die aus Proben von gesunden Subjekten bestimmt wurden.

3. Verfahren nach Anspruch 1 oder 2, wobei die Mengen der Varianten entweder durch Massenspektrometrie gemessen werden oder gemessen werden und optional durch Tandem-Massenspektrometrie identifiziert werden.

4. Verfahren nach Anspruch 3, wobei die eine oder mehrere Varianten aus der Probe zwischen Schritten (a) und (b) unter Verwendung eines Antikörpers oder anderer Affinitätsreagenzien, wie Aptamere oder Affikörper, die für das menschliche Cystein-reiche Darmprotein 1 oder seine Varianten spezifisch sind, extrahiert werden.

5. Verfahren nach Anspruch 1 oder 2, wobei die Varianten des menschlichen Cystein-reichen Darmproteins 1 entweder durch eine oder mehrere strukturelle Variationen modifiziert werden, wie beispielsweise mindestens eine der Folgenden: Disulfidbindungs-Bildung, eine oder mehrere Methylgruppen, und Sauerstoffatome, oder durch mindestens eine Mutation modifiziert werden.

## Revendications

1. Procédé pour soit prédire la réponse d'un patient cancéreux à un médicament, soit prédire le pronostic d'un patient cancéreux, comprenant :
a) la mesure de quantités d'un ou de plusieurs variants de la protéine intestinale riche en cystéine 1 (CRIP1) dans un échantillon du patient, les variants ayant un des poids moléculaires suivants : 8419, 8435, 8450 ou 8465 unités de masse atomique, et
b) la comparaison des quantités de l'un ou des plusieurs variants à des quantités de référence déterminées à partir d'échantillons soit de patients répondant, soit de patients ne répondant pas au médicament, respectivement, soit de patients avec un diagnostic différent.

2. Procédé pour diagnostiquer un cancer chez un sujet, comprenant :
a) la mesure de quantités d'un ou de plusieurs variants de la protéine intestinale riche en cystéine 1 (CRIP1) dans un échantillon du sujet choisi dans le groupe constitué du sérum, du plasma, du sang entier, du fluide d'aspiration du mamelon, du fluide cérébrospinal et de l'urine, les variants ayant un des poids moléculaires suivants : 8419, 8435, 8450 ou 8465 unités de masse atomique, et
b) la comparaison des quantités de l'un ou des plusieurs variants à des quantités de référence déterminées à partir d'échantillons de sujets sains.

3. Procédé selon la revendication 1 ou 2, dans lequel les quantités des variants sont soit mesurées par spectrométrie de masse, soit mesurées et facultativement identifiées par spectrométrie de masse tandem.

4. Procédé selon la revendication 3, dans lequel l'un ou les plusieurs variants sont extraits de l'échantillon entre les étapes a) et b) en utilisant un anticorps ou d'autres réactifs d'affinité, comme des aptamères ou des molécules affibody spécifiques de la protéine intestinale riche en cystéine 1 ou de ses variants.

5. Procédé selon la revendication 1 ou 2, dans lequel les variants de la protéine intestinale riche en cystéine 1 sont modifiés soit par une ou plusieurs variations structurelles, comme au moins un entre la formation d'un pont disulfure, un ou plusieurs groupes méthyle et des atomes d'oxygène, soit par au moins une mutation.
